(19) Europäisches Patentamt
European Patent Office
Office européen des brevets

(11) Numéro de publication: 0 002 164
B1

(12) FASCICULE DE BREVET EUROPEEN

(21) Numéro de dépôt: 78420014.9

(22) Date de dépôt: 14.11.78

(51) Int. Cl.³: C 07 D 309/34

# (54) Procédé de préparation de sels de pyrylium

(30) Priorité: 18.11.77 FR 7735292

(43) Date de publication de la demande:
30.05.79 Bulletin 79/11

(45) Mention de la délivrance du brevet:
26.11.80 Bulletin 80/24

(84) Etats contractants désignés:
BE CH DE FR GB LU NL SE

(56) Documents cités:
JOURNAL OF THE CHEMICAL SOCIETY (1961), pages 3553—3564. 696. Pyrylium salts obtained by diacylation of olefins. Part II.

(73) Titulaire: RHONE-POULENC INDUSTRIES
22, avenue Montaigne
F - 75008 Paris (FR)

(72) Inventeur: Arnaud, Michel
Résidence Le Gounod Boulevard du Bassin
F - 13014 Marseille (FR)
Roussel, Christian
69, boulevard de la Libération
F - 13001 Marseille (FR)

(74) Mandataire: Rioufrays, Roger, et al
RHONE-POULENC INDUSTRIES Centre de Recherches des Carrières Service Brevets
F - 69190 Saint-Fons (FR)

EP 0 002 164 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Procédé de préparation de sels de pyrylium

La présente invention a pour objet un procédé de préparation de sels de pyrylium comportant au moins trois restes alcoyles en position 2, 4 et 6 par réaction d'un agent d'acylation avec un alcane ramifié en présence d'un acide de Lewis.

Les sels de pyrylium représentés par la formule générale:

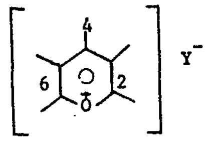

dans laquelle Y représente un anion quelconque, sont des composés présentant un très grand intérêt industriel à des titres divers. Ainsi ils sont utilisés comme adjuvants dans les compositions photosensibles employées en photographie (cf. brevet français n° 1 387 433 et brevet américan n° 3 250 615). En outre les sels de pyrylium constituent des intermédiaires recherchés en synthèse organique. C'est ainsi que par réaction avec l'ammoniac ou les amines les sels de pyrylium conduisent aux bases pyridiniques correspondantes. Par action d'une base alcaline telle que la soude, les sels de pyrylium trisubstitués comportant un groupe méthyle en position 2 sont transformés en phénols disubstitués. En dehors de ces réactions particulièrement importantes du point de vue industriel, les sels de pyrylium reçoivent de nombreuses autres applications en synthèse organique (cf. S. V. KRIVUN et al., Russ. Chem. Rev. *43* 835—850 [1974]).

La synthèse des sels de pyrylium a fait l'objet de nombreux travaux (cf. A. T. BALABAN Advances in Heterocyclic Chemistry vol. 10 pages 241—326 [1969]). Parmi les divers procédés de préparation des sels d'alcoyl pyrylium proposés, les plus intéressants industriellement sont ceux qui mettent en oeuvre la diacylation des oléfines ou de leurs précurseurs tels que les halogénures d'alcoyles tertiaires, les alcools tertiaires ou secondaires ou leurs esters, en présence d'acides de Lewis comme les halogénures le divers métaux ($AlCl_3$, $FeCl_3$, $SbCl_5$, $SnCl_4$, $ZnCl_2$ par exemple) ou le trifluorure de bore ou ses complexes avec les oxydes d'alcoyle (oxyde d'éthyle, d'isopropyle) ou encore en présence d'un acide de Bronsted fort comme l'acide perchlorique ou l'acide trifluorométhane sulfonique. Comme agents d'acylation on utilise les anhydrides d'acides et plus particulièrement les halogénures d'acides aliphatiques ou aromatiques. La diacylation des oléfines peut être représentée schématiquement de la façon suivante:

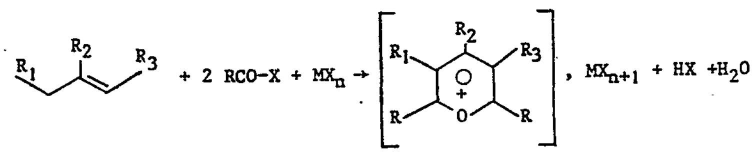

Les rendements en sels de pyrylium varient suivant l'oléfine (ou son précurseur), l'agent d'acylation et le catalyseur utilisé (cf. A. T. BALABAN et al., Ann. *625* pages 74 à 88 [1959]; J. Chem. Soc. *1961* pages 3553—3561; brevets français n° 1 340 970 et 1 340 971).

En dépit de l'intérêt que présente la diacylation des oléfines, il apparaît souhaitable de point de vue industriel de disposer d'un procédé de préparation des sels de pyrylium â partir des alcanes ramifiés ou de leurs mélanges tels que les coupes d'hydrocarbures aliphatiques saturés disponibles en quantités industrielles. Il est surprenant de constater que bien que la diacylation des oléfines ou de leurs précurseurs ait fait l'objet de nombreux travaux depuis 1959, on ne trouve aucune publication sur la préparation des sels de pyrylium à partir des alcanes ramifiés. H. HOPFF et al. (Ber. *69* pages 2244 à 2251 [1936]) ont bien étudié la réaction du chlorure d'acétyle avec des hydrocarbures saturés, et notamment avec l'isobutane, mais ils ne font pas état de la formation de sels de pyrylium.

La présente invention a précisément pour objet un procédé de préparation de sels de pyrylium alcoylsubstitués à partir des alcanes ramifiés.

Plus particulièrement la présente invention a pour objet un procédé de préparation de sels de pyrylium comportant au moins trois substituants alcoyles en position 2, 4, 6 à partir d'un alcane ramifié comportant au moins un atome de carbone tertiaire, caractérisé en ce que l'on fait réagir ledit alcane avec un agent acylant dérivé d'un acide alcanoîque et un halogénure d'alcoyle, en présence d'au moins un acide de Lewis pris dans le groupe formé par les halogénures d'aluminium et les halogénures ferriques.

Plus spécifiquement la présente invention a pour objet un procédé de préparation des sels de pyrylium de formule générale:

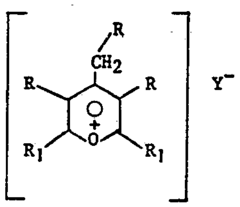

(I)

dans laquelle:

— les différents radicaux R qui peuvent être identiques ou différentes représentent un atome d'hydrogène, un radical alcoyle linéaire ou ramifié comportant de 1 à 10 atomes de carbone,

— $R_1$ représente un radical alcoyle linéaire ou ramifié ayant de 1 à 10 atomes de carbone,

— $Y^-$ représente un anion dérivé d'un acide minéral ou organique, par réaction d'un alcane ramifié de formule:

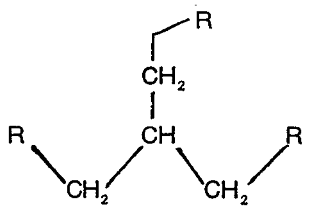

(II)

dans laquelle les radicaux R ont la signification donnée ci-avant, avec un agent acylant de formule générale:

$$R_1 - CO - Z \qquad (III)$$

dans laquelle Z représente un atome d'halogène tel que le chlore et le brome ou un reste de formule générale:

$$R_1 - CO - O \qquad (IV)$$

et les radicaux $R_1$ des formules (III) et (IV) ont la même signification que celle donnée pour la formule (I).

Comme exemples de radicaux R on peut citer les radicaux méthyle, éthyle, n-propyle, n-hexyle- n-heptyle, n-décyle, isopropyle, diméthyl-2,2 propyle; $R_1$ peut être un radical méthyle, éthyle, isopropyle, n-butyle, sec-butyle, t-butyle, pentyles, hexyles, décyles.

Les radicaux $R_1$ représentent de préférence des radicaux alcoyles ayant de 1 à 5 atomes de carbone.

D'une manière générale Y peut représenter un anion dérivé d'un acide minéral ou organique. Comme exemples d'anions minéraux on peut citer à titre non limitatif les ions chlorure, bromure, iodure, sulfate, nitrate, perchlorate, trifluoroborate, tétrahalogénoaluminate, tétrahalogénoferrate tels que les tetrafluoroaluminate, les tétrachloro-, tétrabromoaluminates, le tétrachloroferrate, les halogénozincates, -titanates, -antimoniates, -stannates; comme exemples d'anions dérivés d'acides organiques on peut citer notamment les sulfonates tels que les benzènesulfonate, toluènesulfonate, éthanesulfonate, trifluorométhanesulfonate.

Parmi les agents acylants de formule (III) on peut faire appel à des halogénures d'alcanoyles tels que le chlorure d'acétyle, le bromure d'acétyle, le chlorure de propionyle, le chlorure de n-butyryle, le chlorure de n-pentanoyle, le chlorure d'isobutyryle, le bromure d'isovaléryle, le chlorure de pivaloyle; on peut également utiliser les anhydrides tels que l'anhydride acétique, l'anhydride propionique ou l'anhydride butyrique. Les halogénures d'acides constituent cependant les agents d'acylation préférés.

L'halogénure d'alcoyle utilisé dans le procédé selon l'invention peut comporter un ou plusieurs halogènes, avec le restriction que dans ce dernier cas les atomes d'halogènes ne soient pas portés par le même atome de carbone. On peut les représenter par la formule générale:

$$R_2 - X \qquad (V)$$

dans laquelle $R_2$ représente un radical alcoyle linéaire ou ramifié ayant de 1 à 20 atomes de carbone, éventuellement substitué par un ou plusieurs atomes d'halogènes avec la restriction déjà indiquée; X représente un atome d'halogène: iode; fluor, chlore, brome; de préférence X est un atome de chlore ou de brome.

Parmi les halogénures d'alcoyle mis en oeuvre dans le procédé de l'invention on peut citer: le chlorure de méthyle, le bromure de méthyle, le chlorure d'éthyle, le bromure d'éthyle, le dichloro-1,2 éthane, le chlorure de n-propyle, le chlorure et le bromure d'isopropyle, les chlorures et les bromures de n-butyle, d'isobutyle, de t-butyle, de sec-butyle, de n-pentyle, de t-amyle, de méthyl-3 butyle, de

diméthyl-2,3 butyle, de n-hexyle, de n-octyle, d'éthyl-2 hexyle, de n-décyle. On fait appel de préférence aux monochlorures et monobromures d'alcoyle ayant de l à 10 atomes de carbone.

Parmi les alcanes ramifiés de formule (II) auxquels on peut faire appel pour préparer les sels de pyrylium on peut citer à titre d'exemples non limitatifs: l'isobutane, l'isopentane, le méthyl-2 pentane, le méthyl-3 pentane, le méthyl-2 hexane, l'isooctane (triméthyl-2,2,4 pentane), le diméthyl-2,3 butane.

Parmi les alcanes ramifiés précités, on utilise de préférence les isoalcanes, c'est-à-dire les alcanes de formule (II) dans laquelle les duex radicaux R situés sur les atomes de carbone en position 1 et 3 da la formule (II) représentent des radicaux alcoyles identiques, le radical R porté par l'atome de carbone en position 2 représentant un groupe alcoyle linéaire ou ramifié. Les isoalcanes ayant de 4 à 10 atomes de carbone conviennent tout particulièrement bien.

Sans que la portée de l'invention soit limitée en quoi que ce soit à un mécanisme particulier, la réaction peut être représentée dans le cas où l'agent acylant est un halogénure d''alcanoyle par le schéma suivant:

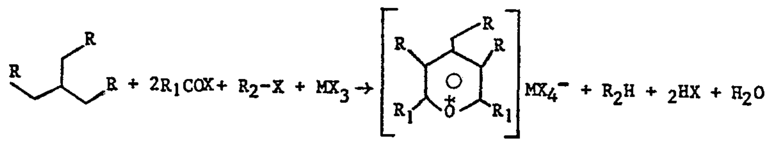


On constate que l'halogénure d'alcoyle est réduit au cours de la réaction en l'alcane correspondant lequel peut être utilisé pour préparer l'halogénure d'alcoyle de départ par les procédés usuels. On a observé avec surprise que lorsque l'halogénure d'alcoyle est un halogénure tertiaire tel que le chlorure de t-butyle ou le chlorure d'isoamyle la diacylation de ce dernier, conformément à l'enseignement de la littérature, ne se produit pratiquement pas en présence de l'alcane ramifié.

On a encore constaté que dans le cas où deux au moins des radicaux R sont différents, la réaction aboutit à la formation d'un mélange de sels de pyrylium. Ainsi, au départ d'un alcane ramifié de formule générale:

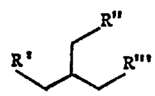 (VI)

dans laquelle les radicaux R', R'' et R''' sont différents, on peut obtenir les composés suivants

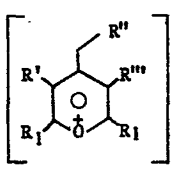 $Y^-$ et/ou 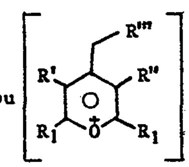 $Y^-$ et/ou 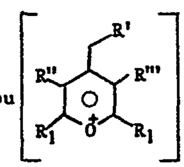 $Y^-$

Lorsqu'on opère au départ d'isoalcanes de formule générale:

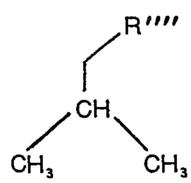

la réaction conduit à la formation des sels de pyrylium isomères:

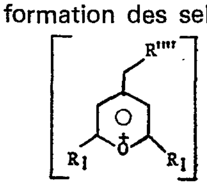 $Y^-$ et 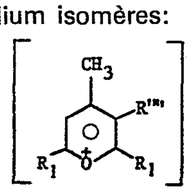 $Y^-$

Pour un alcane ramifié donné, les quantités respectives de sels de pyrylium isomères formés dépendent des conditions de la réaction, par exemple de la quantité de l'acide de Lewis, d'agent acylant et de la température, de sorte que l'on peut agir sur la formation des sels isomères par un choix judicieux de ces divers facteurs.

Les quantités respectives d'alcane ramifié et d'agent acylant peuvent varier dans de larges limites et généralement on peut utiliser un excès de l'un ou de l'autre des réactifs par rapport aux proportions molaires théoriquement nécessaires. Ainsi on pourrait mettre en oeuvre de 0,1 à 10 moles d'alcane par mole d'agent d'acylation. Toutefois il est préférable d'opérer en présence d'une quantité d'alcane au moins égale à celle théoriquement nécessaire pour réaliser la diacylation. Ainsi on met en oeuvre de préférence au moins 0,5 mole d'alcane par mole d'agent acylant, et en particulier de 0,5 à 2 moles d'alcane par mole d'agent acylant.

La quantité d'halogénure d'alcoyle mise en oeuvre dépend de sa nature. Ainsi lorsque l'halogénure d'alcoyle est un halogénure primaire quelconque, on peut utiliser un large excès par rapport à la quantité théoriquement nécessaire; il n'y a pas alors de limite supérieure critique et la quantité maximale est déterminée par des considérations d'ordre pratique. D'une manière générale, la quantité d'halogénure d'alcoyle peut être comprise entre 0,1 et 10 moles par mole d'alcane. Au contraire lorsque l'halogénure d'alcoyle est susceptible de subir par lui-même, dans les conditions de la réaction, une diacylation pour conduire aux sels de pyrylium (cas des halogénures d'alcoyle tertiaires et des halogénures d'alcoyle secondaire), il est préférable de ne pas utiliser un trop fort excès d'halogénure d'alcoyle par rapport à l'alcane ramifiè. On utilise alors une quantité d'halogènure d'alcoyle voisine de la quantité stoechiométrique, c'est-à-dire voisine d'une mole d'halogénure d'alcoyle par mole d'alcane ramifié. De préference dans ce cas la quantité d'halogénure tertiaire ou secondaire est comprise entre 0,9 et 1,1 mole par mole d'alcane.

Que les halogénures d'aluminium ou de fer soient utilisés seuls ou en mélange entre eux, la quantité globale de catalyseur exprimée par le nombre de moles d'acide de Lewis par mole d'agent acylant dépend de la nature de ce dernier. En règle générale on met en oeuvre une quantité de catalyseur voisine de la quantité steochiométrique du système agent acylant/catalyseur considéré; on peut faire appel à un excès de catalyseur par rapport à la quantité stoechiomètrique mais cela ne se traduit pas par un avantage particulier. Il n'y a pas d'avantage non plus à mettre en oeuvre une quantité d'acide de Lewis trop faible par rapport à la quantité théoriquement nécessaire pour le système considéré. En général une quantité totale d'acide de Lewis comprise entre 0,2 et 2,5 moles par mole d'agent acylant convient bien; de préférence cette quantité est comprise entre 0,4 et 1,5 mole par mole d'agent acylant.

Lorsque l'on fait appel à un mélange d'halogénure d'aluminium et d'halogénure ferrique pour mettre en oeuvre le procédé selon la présente invention, les quantités respectives des constituants du mélange peuvent varier dans le larges limites. Ces proportions sont déterminés en fonction de l'influence que chacun des constituants exerce sur la sélectivité de la réaction en les divers sels de pyrylium. Cette influence peut être établie dans chaque cas au moyen d'essais simples. En général quand on utilise un mélange d'halogénure d'aluminium et d'halogénure ferrique les proportions molaires de chacun d'entre eux dans le mélange peuvent varier entre 0,1 et 99,9%.

La température à laquelle la réaction est conduite peut varier entre 0 et 100°C et de préférence entre 15 et 80°C. En général il n'est pas nécessaire de dépasser des températures de 50°C. On conduit de préférence la réaction à une température comprise entre 15 et 30°C. Bien qu'il ne soit généralement pas nécessaire d'opérer sous pression, il peut être avantageux lorsqu'un des réactifs est gazeux dans les conditions de la réaction de conduire cette dernière sous pression autogène.

Le procédé selon l'invention peut être mis en oeuvre en absence de tout solvant ou en présence d'un solvant organique inerte dans les conditions de la réaction. Comme exemples de tels solvants, on peut citer le sulfure de carbone, le tétrachlorure de carbone, le chloroforme, le nitrobenzène, le nitrotoluène, le nitrométhane, le nitroéthane, le nitropropane. La présence de petites quantités d'eau dans le mileur réactionnel n'est pas gênante et il n'est pas nécessaire de faire appel à des réactifs anhydres. En particulier on peut utiliser comme catalyseurs des halogénures métalliques n'ayant pas subi un traitement propre à les rendre anhydres.

Sur le plan pratique, diverses méthodes peuvent être utilisées pour mettre en oeuvre le procédé selon l'invention. On peut par exemple introduire progressivement dans l'alcane contenant le catalyseur successivement ou simultanément l'halogénure d'alcoyle et l'agent acylant, la température du milieu réactionnel étant maintenue à la valeur choisie. Il est préférable d'ajouter l'halogénure d'alcoyle au mélange de l'alcane, du catalyseur, de l'agent acylant et éventuellement d'un solvant inerte. Dans ce cas on a intérêt à maintenir ce mélange à une température aussi basse que possible pendant l'addition de l'halogénure d'alcoyle. Cette température d'addition est avantageusement comprise entre −10 et 0°C. Lorsque l'introduction de l'halogénure d'alcoyle est achevée la température est portée éventuellement à la valeur choisie la réaction. La durée d'addition de l'halogénure d'alcoyle peut prendre des valeurs très différentes. Il est toutefois préférable que cette addition soit réalisée dans un laps de temps aussi court que possible. Ainsi la durée d'addition est de préférence inférieure à 10 minutes et plus particulièrement à 5 minutes. Lorsqu'un des réactifs est gazeux dans les conditions de la réaction (alcane ramifié ou halogénure d'alcoyle) on peut opérer en autoclave ou faire arriver progressivement le ou les réactifs gazeux dans une solution ou une suspension maintenue à la température adéquate du catalyseur dans un solvant inerte.

Les sels de pyrylium obtenus par le procédé selon l'invention peuvent être séparés du milieu réactionnel par les méthodes connues, par exemple par extraction à l'eau. On obtient alors des solutions aqueuses de sels de pyrylium dont l'anion est la base conjugée de l'acide de Lewis. Pour obtenir des sels de pyrylium dérivés d'autres acides il suffit de traiter la masse réactionnelle ou la solution aqueuse d'extraction au moyen d'un acide tel que les acides perchlorique, sulfurique, chlorhydrique ou sulfonique. Lorsqu'on désire utiliser les sels de pyrylium comme intermédiaires en synthèse organique, par exemple pour la préparation de phénols et de pyridines substitués, il n'est pas nécessaire de les isoler; on peut alors traiter la masse réactionnelle de diacylation des alcanes ramifiés par une base

alcaline pour obtenir les phénols ou par l'ammoniaque ou les amines pour obtenir les bases hétérocycliques, selon les procédés connus.

Le procédé selon l'invention présente un intérêt industriel tout particulier car il permet d'utiliser comme matière première pour la synthèse des sels de pyrylium et éventuellement pour la synthèse de phénols et de pyridines substitués, des mélanges d'alcanes ramifiés et non ramifiés, et en particulier des coupes de paraffines ayant de 5 à 8 atomes de carbone.

Les exemples suivants illustrent l'invention et montrent comment elle peut être mise en pratique. Dans ces exemples les sels de pyrylium ont été identifiés et dosés sous forme des pyridines correspondantes obtenues par traitement de la masse réactionnelle de diacylation par l'ammoniaque.

EXEMPLE 1

Dans un réacteur cylindrique en verre de 400 cm3 refroidi par un bain de glace, muni d'un agitateur du type vibromixeur, d'un réfrigérant ascendant refroidi par circulation d'alcool maintenu à —15°C, d'une ampoule de coulée et d'un thermomètre, on introduit 26,7 g (0,2 mole) de chlorure d'aluminium, puis, en maintenant la température dans l'appareil à 0°C, on charge 36 g (0,5 mole) d'isopentane. On ajoute ensuite goutte à goutte 7,85 g (0,1 mole) de chlorure d'isopropyle puis 23,55 g (0,3 mole) de chlorure d'acétyle. On laisse ensuite la température de mélange réactionnel s'élever à 27°C et maintient sous agitation 2 h 30 mn dans ces conditions. Le dégagement d'acide chlorhydrique est alors terminé.

La masse réactionnelle ainsi obtenue est refroidie à 0°C, puis on ajoute dans l'appareil 250 cm3 d'une solution aqueuse d'ammoniaque à 36% en poids. Les produits organiques formés sont extraits par traitement en continu de la masse réactionnelle par du chloroforme dans un extracteur liquide-liquide. La solution chloroformique est ensuite traitée par une solution aqueuse d'acide chlorhydrique à 10% en poids, puis on sépare la phase aqueuse contenant les dérivés pyridiniques sous forme de chlorhydrate, de la phase chloroformique. La phase aqueuse est neutralisée à la soude et les pyridines sont extraites par traitement en continu de la phase aqueuse par du dichlorométhane dans un extracteur liquide-liquide.

La solution ainsi obtenue est traitée pour évaporer le dichlorométhane. On recueille de cette façon 9,35 g d'un résidu organique que l'on soumet à l'analyse. Les produits ont été identifiés par séparation par chromatographie préparative en phase vapeur puis identification par résonnance magnétique nucléaire et spectrométrie de masse.

De cette façon on a identifié et dosé les composés suivants dans le résidu obtenu:

| PRODUITS | | TENEUR PONDERALE % |
|---|---|---|
| (a) | 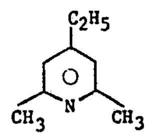 | 35,3 |
| (b) | 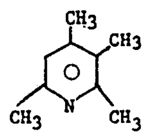 | 41,4 |
| (c) | 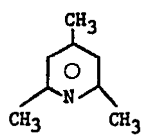 | 1,4 |
| (d) | 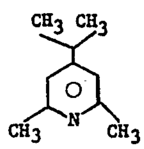 | 1,7 |
| (e) | 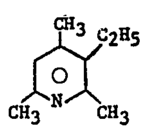 | 1,1 |
| (f) | 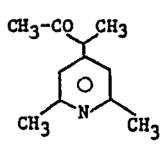 | 2,5 |
| (g) | 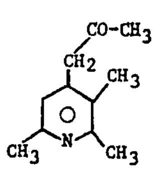 | 16,4 |

ESSAI COMPARATIF 1:

A titre comparatif on a répété l'expérience précédente mais en opérant en absence de chlorure d'isopropyle. On constate dans ces conditions que le dégagement acide chlorhydrique n'a lieu que lorsque la température atteint 35°C. Il faut une durée de réaction de 5 h 30 mn en laissant la température s'élever à 45°C pour voir cesser le dégagement d'acide chlorhydrique.

Après traitement de la masse réactionnelle comme à l'exemple 1 on obtient 7,45 g de mélange pyridinique dans lequel on a identifié et dosé:

| Produits | Pourcentage pondéral |
|---|---|
| (a) | 17,6 |
| (b) | 67,9 |
| (c) | 1,6 |
| (f) | 6,5 |
| (g) | 6,4 |

ESSAI COMPARATIF 2:

On opère en répétant l'expérience précédente mais en remplaçant l'isopentane par le chlorure de t-amyle et en absence de chlorure d'isopropyle. La réaction est terminée après 3 h 30 mn à 30—35°C.

Après traitement de la masse réactionnelle comme précédemment on recueille 3,9 g de mélange pyridinique dans lequel on a dosé et identifié:

| Produits | Pourcentage pondéral |
|---|---|
| (a) | 20,2 |
| (b) | 24,3 |
| (c) | 14,1 |
| (d) | 2,4 |
| (e) | 2,1 |
| (f) | 5,8 |
| (g) | 19,2 |
| (h) 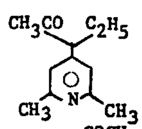 | 2,9 |
| (i) 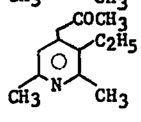 | 1,2 |

Les spectres de résonnance magnétique nucléaire du proton (RMNP) des composés (d) à (i) présentent les caractéristiques suivantes:

— composé (d) dans le tétrachorure de carbone:
doublet à 1,22 ppm; singulet à 2,42 ppm: massif à 2,7 ppm; singulet à 6,67 ppm

— composé (e) dans le chloroforme deutéré:
tripulet à 1,1 ppm; singulet à 2,23 ppm; singulets à 2,5, 2,6 et 6,76 ppm

— composé (f) dans le chloroforme duetéré ($CDCl_3$):
doublet à 1,48 ppm; singulets à 2,08, 2,52 et 6,84 ppm, quadruplet à 3,66 ppm

— composé (g) dans $CDCl_3$:
singulets à: 2,12, 2,17, 2,45, 2,49, 3,68 et 6,76 ppm

— composé (h) dans $CDCl_3$:
triplet à 0,83 ppm; singulets à 2,15, 2,46 et 6,82 ppm
— composé (i) dans $CDCl_3$:
triplet à 1,08 ppm; singulets à 2,16, 2,48, 3,64 et 6,77 ppm; quadruplet masqué à 2,56 ppm.

Dans les exemples qui suivent (a), (b), (c), (d), (e), (f), (g), (h), (i), désigneront les composés pyridiniques dont les formules figurent dans le présent exemple.

## EXEMPLE 2

On opère comme à l'exemple 1 mais en remplaçant le chlorure d'aluminium par 32,5 g de chlorure ferrique et le chlorure d'isopropyle par 9,25 g de chlorure de t-butyle.

Dans ces conditions on a obtenu après traitement de la masse réactionnelle comme à l'exemple 1, 9,3 g de mélange pyridinique dans lequel on a dosé et identifié:

| | | |
|---|---|---|
| (a) | 60,8% | en poids |
| (b) | 17,4% | „ |
| (c) | 4,1% | „ |
| (f) | 0,8% | „ |
| (g) | 16,7% | „ |

## EXEMPLE 3

On opère comme à l'exemple 1, mais en remplaçant l'isopentane par 43 g (0,5 mole) de méthyl-3 pentane et en ajoutant successivement le chlorure d'acétyle puis le chlorure d'isopropyle lorsque la température atteint 15°C. La masse réactionnelle est maintenue 3 h 30 mn à 28°C. Après traitement de la masse réactionnelle comme à l'exemple 1 on recueille 7,6 g de mélange pyridinique dans lequel on a identifié et dosé:

| | | |
|---|---|---|
| (j) | 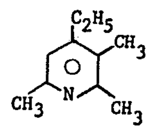 | 13,3% en poids |
| (k) | 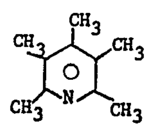 | 34,9% en poids |
| (c) | | 0,7% en poids |
| (a) | | 0,4% en poids |
| (d) | | 1,2% en poids |
| (e) | | 31,3% en poids |
| (1) | 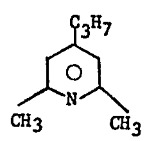 | 11,8% en poids |
| (h) | | 1,1% en poids |
| (m) | 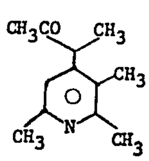 | 2,4% en poids |
| (i) | | 1,9% en poids |
| (n) | 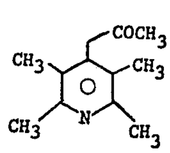 | 0,9% en poids |

ESSAI COMPARATIF

A titre comparatif on a répété l'expérience précédente en opérant en l'absence de chlorure d'isopropyle. Les quantités de réactifs ont été les suivantes:

| | |
|---|---|
| — méthyl-3 pentane | 86 g |
| — chlorure d'acétyle | 40 g |
| — chlorure d'aluminium | 46,7 g |

La durée de la réaction a été de 4 h à 44°C.

Après traitement de la masse réactionnelle on a recueilli 8,8 g de mélange pyridinique dans lequel on a dosé et identifié les produits suivants:

| | |
|---|---|
| (c) | 0,5% en poids |
| (d) | 0,3% " |
| (e) | 43,5% " |
| (l) | 8,2% " |
| (h) | 6,6% " |
| (m) | 6,2% " |
| (i) | 10,7% " |
| (n) | 3,2% " |
| (j) | 7 % " |
| (k) | 12,8% " |

Les spectres de résonnance magnétique nucléaire du proton des composés (j) à (n) présentent les caractéristiques suivantes:

— composé (j) dand $CDCl_3$:
triplet à 1,16 ppm; singulet à 2,16, 2,48 et 6,67 ppm; quadruplet à 2,56 ppm

— composé (k) dans $CDCl_3$:
singulets à 2,2 et 2,5 ppm

— composé (1) dans $CDCl_3$:
triplet à 0,89 ppm; massif à 1,57 ppm singulet et triplet à 2,45 ppm; singulet à 6,74 ppm

— composé (m) dans $CDCl_3$:
doublet à 1,31 ppm; singulets à: 2; 2,2; 2,43; 2,46 et 6,7 ppm; quadruplet à 3,9 ppm

— composé (n) dans $CDCl_3$:
singulets à: 2,14; 2,22; 2,5 et 3,82 ppm.

EXEMPLE 4

On opère comme à l'exemple 3 mais en remplaçant le méthyl-3 pentane par le méthyl-2 pentane. La réaction dure 2 h 30 mn à 30°C.

On a recueilli 6,7 g de mélange pyridinique dans lequel on a dosé:

| | |
|---|---|
| (d) | 0,5% en poids |
| (e) | 32 % „ |
| (j) | 7 % „ |
| (k) | 18 % „ |
| (l) | 17 % „ |
| (h) | 5 % „ |
| (m) | 7 % „ |
| (i) | 10 % „ |
| (n) | 6 % „ |

EXEMPLE 5

On opère comme à l'exemple 4 en remplaçant le mèthyl-2 pentane par le diméthyl-2,3 butane. On a recueilli 5,6 g de mélange pyridinique dans lequel on a dosé et identifié:

| | | |
|---|---|---|
| (c) | | 0,8% en poids |
| (d) | | 37,6% „ |
| (e) | | 1,3% „ |
| (j) | | 2,1% „ |
| (k) | | 1,1% „ |
| (l) | | 0,9% „ |
| (m) | | 1,3% „ |
| (i) | | 1,4% „ |
| (o) | 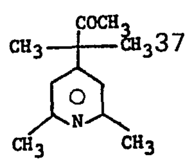 | 37 % „ |

Le composé (o) présente un spectre RMNP ayant les caractéristiques suivantes: singulets à: 1,43; 1,92; 2,5 et 6,83 ppm.

EXEMPLE 6

Dans un réacteur en verre tell que celui décrit à l'exemple 1, on charge 53,4 g (0,4 mole) de chlorure d'aluminium.

En maintenant la température à 0°C, on ajoute 14,4 g (0,2 mole) d'isopentane dans 50 g de chloroforme puis 31,4 g (0,4 mole) de chlorure d'acétyle. On porte la température á 30°C, puis on ajoute goutte à goutte 15,7 g (0,2 mole) de chlorure d'isopropyle dans 50 g de chloroforme. Un dégagement d'acide chlorhydrique se produit rapidement et cesse après 3 heures dans ces conditions. On maintient le mélange réactionnel à 30°C pendant encore 12 heures. Le milieu réactionnel est un liquide jaune foncé et limpide. On verse alors environ 200 cm3 d'ammoniaque (d = 0,92) avec précautions au début car la réaction est très vive (on refroidit avec un bain de glace). On obtient alors une phase organique et une phase aqueuse contenant la précipité compact des sels d'aluminium. On verse ce mélange dans un extracteur liquide-liquide d'environ 800 cm3. On lave le réacteur avec environ 200 cm3 d'ammoniaque et du chloroforme et ajoute le liquide de lavage au contenu de l'extracteur. On extrait en continu, avec 300 cm3 environ de chloroforme (duréede l'extraction

6 heures) les produits organiques issus de la réaction. Après évaporation du chloroforme, on traite le mélange de produits organiques par environ 150 cm3 d'acide chlorhydrique à 10% (jusqu'a pH = 1). On sépare les phases aqueuses et organiques. On lave la phase aqueuse par environ 20 cm3 de chloroforme. La phase aqueuse est traitée par un excès de soude en pastilles jusqu'à pH = 12, puis les pyridines sont extraites en continu par environ 150 cm3 de dichlorométhane dans un extracteur de 300 cm3. On évapore ensuite le dichlorométhane et obtient un résidu pesant 10,7 g dans lequel on a identifié et dosé:

| | | |
|---|---|---|
| (a) | 0.96% | en poids |
| (b) | 94 % | en poids |
| (c) | 1,4 % | en poids |
| (g) | 0,6 % | en poids. |

EXEMPLE 7

Dans un réacteur cylindrique en verre, équipé d'une double enveloppe, d'une agitation par vibromixeur et d'un réfrigérant ascendant, on dispose 200 g de chloroforme anhydre fraîchement distillé puis 0,2 mole (26,7 g) de chlorure d'aluminium. On fait circuler dans la double enveloppe de l'eau à 0°C et dans le réfrigérant ascendant de l'alcool à −40°C. On met alors en route l'agitation. On ajoute 0,2 mole de chlorure d'acétyle puis 0,1 mole de méthyl-2 pentane (8,6 g). Toujours à 0°C, on ajoute 0,1 mole (7,85 g) de chlorure d'isopropyle à l'aide d'une burette à piston de façon que l'addition dure 2 mn, puis on porte la température du milieu réactionnel à 25°C et on laisse réagir 2 heures.

On refroidit le milieu réactionnel (circulation d'eau à 0°C) puis on le verse dans une fiole conique en verre contenant 500 ml d'ammoniaque à 34% et environ 100 g de glace en maintenant une agitation efficace. On extrait en continu au chloroforme les produits de la réaction. Les pyridines sont séparées du mélange par un traitement à l'acide chlorhydrique à 5%. La phase aqueuse contenant les sels de pyridinium est traitée par un excès de soude en pastilles, puis les pyridines sont extraites en continu au chlorure de méthylène.

Après évaporation du solvant, le mélange de pyrdidines est séché au dessiccateur sur de la soude, puis pesé et analysé par chromatographie en phase gazeuse.

Le chloroforme contenant les produits non pyridiniques est évaporé et ce résidu est séché sur chlorure de calcium au dessicateur, puis pesé.

On recueille de cette façon 4,1 g de résidu et 5,1 g d'un mélange de pyridines qui ont été identifiées et dosées comme à l'exemple 1. Les composés suivants ont été identifiés:

| | |
|---|---|
| (e) | 86,5% |
| (l) | 4,4% |
| (j) | 1,6% |

diméthyl-2,6 pyridine: 3,5%.

On constate que les produits issus directement de la diacylation du méthyl-2 pentane [(e) et (1)] constituent 90,9% des produits pyridiniques.

**Revendications**

1. Procédé de préparation de sels de pyrylium comportant au moins 3 substituants alcoyles en position 2, 4, 6 à partir d'un alcane ramifié comportant au moins un atome de carbone tertiaire, caractérisé en ce que l'on fait réagir iedit alcane avec un agent acylant dérivé d'un acide alconîque, et un halogénure d'alcoyle, en présence d'au moins un acide de Lewis pris dans le groupe formé par les halogénures d'aluminium et les halogéures ferriques.

2. Procédé selon la revendication 1, caractérisé en ce que l'on prépare des sels de pyrylium de formule générale

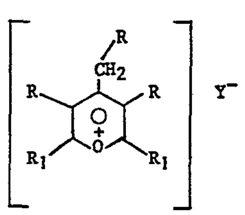

(I)

dans laquelle

— les radicaux R qui peuvent être identiques ou différents représentent un atome d'hydrogène ou un radical alcoyle linéaire ou ramifié comportant de 1 à 10 atomes de carbone,

— $R_1$ représente un radical alcoyle linéaire ou ramifié ayant de 1 à 10 atomes de carbone,

— $Y^-$ représente un anion dérivé d'un acide minéral ou organique, par réaction d'un alcane ramifié de formule générale

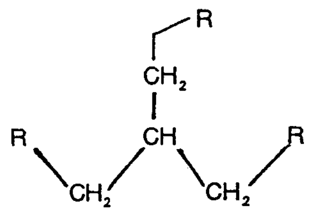

(II)

dans laquelle R a la signification donnée ci-avant, avec un agent acylant de formule générale:

$$R_1 - CO - Z \qquad (III)$$

dans laquelle:

— Z représente un atome d'halogène ou un reste de formule générale:

$$R_1 - CO - O \qquad (IV)$$

— $R_1$ a dans les formules (III) et (IV) la signification donnée pour la formule (I) et avec un halogènure d'alcoyle de formule générale:

$$R_2 - X \qquad (V)$$

dans laquelle:

— $R_2$ représente un radical alcoyle linéaire ou ramifié ayant de 1 à 20 atomes de carbone, éventuellement substitué par 1 ou plusieurs atomes d'halogène, avec la restriction que les divers halogènes des composés de formule (V) ne soient pas portés par le même atome de carbone,

— X représente un atome d'halogène,

en présence d'un halogénure d'aluminium et/ou d'un halogénure ferrique.

3. Procédé selon la revendication 2, caractérisé en ce que l'on utilise un alcane ramifié de formule (II) dans laquelle les radicaux R situés en position 1, 3 sont identiques.

4. Procédé selon l'une quelconque des revendications 1 à 3, caractérisé en ce que l'alcane ramifié est l'isopentane, le méthyl-2-pentane, le méthyl-3 pentane, le diméthyl-2,3 butane.

5. Procédé selon l'une quelconque des revendications 1 à 4, caractérisé en ce que l'agent acylant est un chlorure ou un bromure d'alcanoyle.

6. Procédé selon la revendication 5, caractérisé en ce que l'agent acylant est le chlorure d'acétyle.

7. procédé selon l'une quelconque des revendications 1 à 6, caractérisé en ce que l'halogénure d'alcoyle est un chlorure ou un bromure d'alcoyle.

8. Procédé selon l'une quelconque des revendications 1 à 7, caractérisé en ce que l'halogénure d'alcoyle est le chlorure d'isopropyle, le chlorure de t-butyle.

9. Procédé selon l'une quelconque des revendications 1 à 8, caractérisé en ce que l'acide de Lewis est le chlorure d'aluminium et le chlorure ferrique.

10. Procédé selon l'une quelconque des revendications 1 à 9 caractérisé en ce que la réaction est conduite dans un solvant organique inerte.

11. Procédé selon la revendication 10, caractérisé en ce que la réaction est conduite dans le chloroforme.

12. Procédé selon l'une quelconque des revendications 1 à 11, caractérisé en ce que la quantité d'alcane mise en oeuvre est comprise entre 0,1 et 10 moles par mole d'agent acylant.

13. Procédé selon l'une quelconque des revendications 1 à 12, caractérisé en ce que la quantité d'halogénure d'alcoyle est d'au moins 0,1 mole par mole d'alcane ramifié.

14. Procédé selon l'une quelconque des revendications 1 à 13, caractérisé en ce que la quantité d'acide de Lewis est comprise entre 0,2 et 2,5 mole par mole d'agent acylant.

15. Procédé selon l'une quelconque des revendications 1 à 14, caractérisé en ce que la température de la réaction est comprise entre 0 et 100°C.

16. Procéde selon l'une quelconque des revendications 1 à 15, caractérisé en ce que l'anion $Y^-$ est le tétrachloroaluminate et le tétrachloroferrate.

**Claims**

1. Process for the preparation of pyrylium salts possessing at least 3 alkyl substituents in the 2-, 4- and 6-positions, from a branched alkane possessing at least one tertiary carbon atom, characterised

in that the said alkane is reacted with an acylating agent derived from an alknoic acid, and an alkyl halide, in the presence of at least one Lewis acid selected from the group consisting of the aluminium halides and the ferric halides.

2. Process according to Claim 1, characterised in that pyrylium salts of the general formula

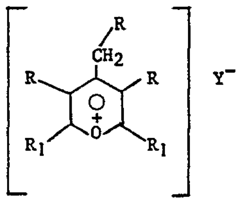

(I)

in which

the radicals R, which may be identical or different, represent a hydrogen atom or a linear or branched alkyl radical possessing from 1 to 10 carbon atoms,

$R_1$ represents a linear or branched alkyl radical having from 1 to 10 carbon atoms and

$Y^-$ represents an anion derived from a mineral acid or organic acid,

are prepared by reacting a branched alkane of the general formula

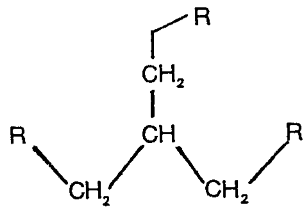

(II)

in which R has the meaning given above, with an acylating agent of the general formula

$$R_1 - CO - Z \quad (III)$$

in which

Z represents a halogen atom or a radical of the general formula

$$R_1 - CO - O \quad (IV)$$

$R_1$ has the same meaning in formulae (III) and (IV) as that given for formula (I), and with an alkyl halide of the general formula:

$$R_2 - X \quad (V)$$

in which

$R_2$ represents a linear or branched alkyl radical having from 1 to 20 carbon atoms, optionally substituted by 1 or more halogen atoms, with the proviso that the various halogens of the compounds of the formula (V) should not be carried by the same carbon atom, and

X represents a halogen atom,

in the presence of an aluminium halide and/or of a ferric halide.

3. Process according to Claim 2, characterised in that a branched alkane of the formula (II), in which the radicals R located in the 1- and 3-positions are identical, is used.

4. Process according to any one of Claims 1 to 3, characterised in that the branched alkane is isopentane, 2-methyl-pentane, 3-methyl-pentane or 2,3-dimethyl-butane.

5. Process according to any one of Claims 1 to 4, characterised in that the acylating agent is an alkanoyl chloride or bromide.

6. Process according to Claim 5, characterised in that the acylating agent is acetyl chloride.

7. Process according to any one of Claims 1 to 6, characterised in that the alkyl halide is an alkyl chloride or an alkyl bromide.

8. Process according to any one of Claims 1 to 7, characterised in that the alkyl halide is isopropyl chloride or tert.-butyl chloride.

9. Process according to any one of Claims 1 to 8, characterised in that the Lewis acid is aluminium chloride or ferric chloride.

10. Process according to any one of Claims 1 to 9, characterised in that the reaction is carried out in an inert organic solvent.

11. Process according to Claim 10, characterised in that the reaction is carried out in chloroform.

12. Process according to any one of Claims 1 to 11, characterised in that the amount of alkane employed is between 0,1 and 10 mols per mol of acylating agent.

13. Process according to any one of Claims 1 to 12, characterised in that the amount of alkyl halide is at least 0.1 mol per mol of branched alkane.

14. Process according to any one of Claims 1 to 13, characterised in that the amount of Lewis acid is between 0.2 and 2.5 mols per mole of acylating agent.

15. Process according to any one of Claims 1 to 14, characterised in that the reaction temperature is between 0 and 100°C.

16. Process according to any one of Claims 1 to 15, characterised in that the anion $Y^-$ is tetrachloroaluminate or tetrachloroferrate.

**Patentansprüche**

1. Verfahren zur Herstellung von Pyryliumsalzen, die wenigstens 3 Alkylsubstituenten in der 2,4,6-Stellung enthalten, aus einem verzweigten Alkan, das wenigstens ein tertiäres Kohlenstoffatom enthält, dadurch gekennzeichnet, daß man das Alkan mit einem von einer Alkansäure abgeleiteten Acylierungsmittel und einem Alkylhalogenid in Gegenwart wenigstens einer Lewis-Säure aus der aus Aluminiumhalogeniden und Eisen(III)-halogeniden bestehenden Gruppe umsetzt.

2. Verfahren nach Anspruch 1, dadurch gekennzeichnet, daß man Pyryliumsalze der allgemeinen Formel:

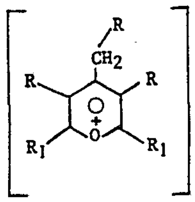

(I)

in der die Reste R, die gleich oder verschieden sein können, jeweils ein Wasserstoffatom oder ein linearer oder verzweigter Alkylrest mit 1 bis 10 C-Atomen sind, $R_1$ ein linearer oder verzweigter Alkylrest mit 1 bis 10 C-Atom ist und $Y^-$ ein von einer Mineralsäure oder organischen Säure abgeleitetes Anion ist, durch Umsetzung eines verzweigten Alkans der allegemeinen Formel:

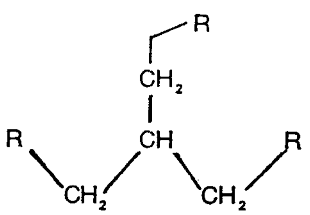

(II)

in der R die vorstehend genannte Bedeutung hat, mit einem Acylierungsmittel der allgemeinen Formel:

$$R_1 - CO - Z \qquad (III)$$

in der Z ein Halogenatom oder ein Rest der allgemeinen Formel:

$$R_1 - CO - O \qquad (IV)$$

ist, wobei $R_1$ in den Formeln (III) und (IV) die für die Formel (I) gennante Bedeutung hat, und mit einem Alkylhalogenid der allgemeinen Formel:

$$R_2 - X \qquad (V)$$

in der $R_2$ ein linearer oder verzweigter Alkylrest mit 1 bis 20 C-Atomen ist, der gegebenenfalls mit Halogenatomen einfach oder mehrfach substituiert ist, mit der Begrenzung, daß die verschiedenen Halogenatome der Verbindungen der Formel (V) nicht an das gleiche C-Atom gebunden sind, und X ein Halogenatom ist, in Gegenwart eines Aluminiumhalogenids und/oder Eisen-(III)-halogenids herstellt.

3. Verfahren nach Anspruch 2, dadurch gekennzeichnet, daß man ein verzweigtes Alkan der Formel (II), in der die in 1,3-Stellung stehenden Reste R gleich sind, verwendet.

4. Verfahren nach Anspruch 1 bis 3, dadurch gekennzeichnet, daß man als verzweigtes Alkan Isopentan, 2-Methylpentan, 3-Methylpentan oder 2,3-Dimethylbutan verwendet.

5. Verfahren nach Anspruch 1 bis 4, dadurch gekennzeichnet, daß man als Acylierungsmittel ein Alkanoylchlorid oder -bromid verwendet.

6. Verfahren nach Anspruch 5, dadurch gekennzeichnet, daß man als Acylierungsmittel Acetychlorid verwendet.

7. Verfahren nach Anspruch 1 bis 6, dadurch gekennzeichnet, daß man als Alkylhalogenid ein Alkylchlorid oder -bromid verwendet.

8. Verfahren nach Anspruch 1 bis 7, dadurch gekennzeichnet, daß man als Alkylhalogenid Isopropylchlorid oder t-Butylchlorid verwendet.

9. Verfahren nach Anspruch 1 bis 8, dadurch gekennzeichnet, daß man als Lewis-Säure Aluminiumchlorid und Eisen(III)-chlorid verwendet.

10. Verfahren nach Anspruch 1 bis 9, dadurch gekennzeichnet, daß man die Reaktion in einem inerten organischen Lösungsmittel durchführt.

11. Verfahren nach Anspruch 10, dadurch gekennzeichnet, daß man die Reaktion in Chloroform durchführt.

12. Verfahren nach Anspruch 1 bis 11, dadurch gekennzeichnet, daß man das Alkan in einer Menge zwischen 0,1 und 10 Mol pro Mol Acylierungsmittel verwendet.

13. Verfahren nach Anspruch 1 bis 12, dadurch gekennzeichnet, daß man das Alkylhalogenid in einer Menge von wenigstens 0,1 Mol pro Mol des verzweigten Alkans verwendet.

14. Verfahren nach Anspruch 1 bis 13, dadurch gekennzeichnet, dâ man die Lewis-Säure in einer Menge zwischen 0,2 und 2,5 Mol pro Mol Acylierungsmittel verwendet.

15. Verfahren nach Anspruch 1 bis 14, dadurch gekennzeichnet, daß man die Reaktion bei einer Temperatur zwischen 0° und 100°C durchführt.

16. Verfahren nach Anspruch 1 bis 15, dadurch gekennzeichnet, daß das Anion $Y^-$ Tetrachloraluminat und Tetrachlorferrat ist.